# EUROPEAN PATENT SPECIFICATION

(11) **EP 4 472 586 B1**
(45) Date of publication and mention of the grant of the patent: **04.02.2026**
(21) Application number: 23714155.1
(22) Date of filing: 03.03.2023
(51) Int. Cl.: A61F 7/00, A61F 7/02

(54) **NIGHTTIME WARMING BLANKET**
NÄCHTLICHES WÄRMETUCH
COUVERTURE CHAUFFANTE POUR USAGE NOCTURNE

(30) Priority: 03.03.2022 CN 202210201110; 02.03.2023 US 202318116562
(43) Date of publication of application: 11.12.2024
(60) Divisional application: 25226009.6
(73) Proprietor: NANHAI NANXIN NON-WOVEN CO. LTD., Jiujiang Town, Nanhai District Foshan, Guangdong 528200 (CN)
(72) Inventor: WU, Dong, Suzhou, Jiangsu 215526 (CN); JIN, Yongji, Suzhou, Jiangsu 215526 (CN); ZHAN, Weiqin, Suzhou, Jiangsu 215526 (CN); FU, Linlei, Suzhou, Jiangsu 215526 (CN)
(74) Representative: Meissner Bolte Nürnberg
(86) International application number: PCT/US2023/014444
(87) International publication number: WO 2023/168040

(56) References cited:
- WO-A1-2014/060792
- WO-A1-2018/004808
- JP-A- 2013 010 337
- JP-A- H03 146 074
- US-A- 4 889 135

## Description

### CROSS-REFERENCE TO RELATED APPLICATIONS

This application claims priority under 35 U.S.C. §119 to Chinese Patent Application No. 202210201110.9 filed March 3, 2022.

### TECHNICAL FIELD

Embodiments of the presently-disclosed invention relate generally to warming blankets, such as for nighttime use, including (i) a perspiration absorptive layer (PAL); (ii) a metal coating layer (MCL); and (iii) a transparent coating layer (TCL), in which the MCL is located directly or indirectly between the PAL and the TCL. Embodiments of the presently-disclosed invention also relate to methods of producing a warming blanket.

### BACKGROUND

Metalized materials, such as metalized blankets, traditionally include a metal coating applied to a base substrate, such as a nonwoven or a film. Such metalized materials, for example, provide a mechanism to by which a user's body heat is significantly retained. In this regard, the metalized materials (e.g., also known as a space blanket, mylar blanket, first aid blanket, safety blanket, thermal blanket, etc.) include a heat-reflective metal coating applied to a thin plastic film or nonwoven. Ideally, the metalized materials reflect around 90% of a user's body heat to mitigate heat loss from the user's body.

US 4889135 A discloses a blanket according to the prior art.

One drawback of some metalized materials relates their lack of breathability and/or flexibility, as well as lack of tailoring for use in specific environments. In this regard, applications of such metalized blankets for the retention of body heat may also desire a desirable level of vapor permeability and/or flexibility (e.g., to easily conform to a user's body).

### SUMMARY OF INVENTION

The invention is defined in independent claims 1 and 15, further embodiments are described in the dependent claims.

### BRIEF DESCRIPTION OF THE DRAWING(S)

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. Like numbers refer to like elements throughout, and wherein:
Figure 1 illustrates a warming blanket in accordance with certain embodiments of the invention;
Figure 2 illustrates a perspiration absorptive layer (PAL) including a plurality of through-holes in accordance with certain embodiments of the invention; and
Figure 3 illustrates the PAL of Figure 2 overlying a metal coating layer (MCL) that is visible through the plurality of through-holes, in accordance with certain embodiments of the invention.

### DETAILED DESCRIPTION

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise.

Certain embodiments of the invention generally relate to warming blankets, such as for use outside and/or during the nighttime, that include a perspiration absorptive layer (PAL), a metal coating layer (MCL), and a transparent coating layer (TCL), in which the MCL is located directly or indirectly between the PAL and the TCL. For example, the TCL may define a first outermost layer of the warming blanket and the PAL may define a second outermost layer of the warming blanket, in which the MCL constitutes at least one layer between the two outermost layers of the warming blanket. The warming blanket, for example, may be particularly suitable for use as a warming system for outside activities in a cold environment and/or during the nighttime (e.g., after sunset). The TCL, for example, provides a layer that mostly transmits several wavelengths of light therethrough and enables the external light (e.g., electromagnetic radiation) to reach the MCL, while in accordance with certain embodiments the TCL also functions as a layer of thermal insulation to mitigate heat loss from the MCL into the external environment. The MCL layer, for example, provides thermal reflection that may reflect electromagnetic radiation from the external environment (e.g., back towards the external environment) and from the body of a user (e.g., back towards the user). The PAL, for example, may comprise a fabric that is capable of absorbing and/or wicking away perspiration from a user's body, which may be particularly desirable as any perspiration that remains on a user's body and/or is allowed to pool against a user's body will eventually cool-off and act as a heat-sink and undesirably pull heat from the body of the user. Additionally, the PAL may comprise a plurality of through-holes that function as windows or unobstructed gateways for electromagnetic radiation leaving a user to strike the MCL and be reflected back to the user to prevent or mitigate a loss of body heat from the user. In use, for example, the PAL may be positioned adjacent or proximate a user, such as a mammal, and the plurality of through-holes enable a high level of generally unobstructed access to the MCL by radiation or heat emitted by a user, in which this radiation or heat is mostly (or all) reflected back to the user by the MCL. In use, that is, the PAL will typically be positioned proximate a user, while the TCL will be positioned distal from the user.

In accordance with certain embodiments of the invention, the warming blanket may be used as a reflective and warming layer to reduce heat loss from a human body. In this regard, the warming blanket may be provided in the form of gowns, facemasks, sterilization wraps, head coverings, heating pads, surgical drape, medical warming blanket, and outing warming blanket applications with high reflectivity, good flexibility, enough pliability and breathability. For example, during cold weather in the wild, wrapping a warming blanket around a user's body can help prevent loss of emitted heat and reduce body heat loss.

In accordance with certain embodiments of the invention, the TCL, and/or, the PAL and/or the warming blanket may comprise a desirable level of flexibility (e.g., as measured by Handle-O-Meter) to provide sufficient drapeability and/or wrapability (e.g., wrapped around a user) and/or desirable breathability (e.g., allow vapors to travel through the warming blanket and out the other side of the warming blanket) and/or desirable level of liquid penetration resistance as measured by hydrostatic head.

The terms "substantial" or "substantially" may encompass the whole amount as specified, according to certain embodiments of the invention, or largely but not the whole amount specified (e.g., 95%, 96%, 97%, 98%, or 99% of the whole amount specified) according to other embodiments of the invention.

The terms "polymer" or "polymeric", as used interchangeably herein, may comprise homopolymers, copolymers, such as, for example, block, graft, random, and alternating copolymers, terpolymers, etc., and blends and modifications thereof. Furthermore, unless otherwise specifically limited, the term "polymer" or "polymeric" shall include all possible structural isomers; stereoisomers including, without limitation, geometric isomers, optical isomers or enantionmers; and/or any chiral molecular configuration of such polymer or polymeric material. These configurations include, but are not limited to, isotactic, syndiotactic, and atactic configurations of such polymer or polymeric material. The term "polymer" or "polymeric" shall also include polymers made from various catalyst systems including, without limitation, the Ziegler-Natta catalyst system and the metallocene/single-site catalyst system. The term "polymer" or "polymeric" shall also include, in according to certain embodiments of the invention, polymers produced by fermentation process or biosourced.

The terms "nonwoven" and "nonwoven web", as used herein, may comprise a web having a structure of individual fibers, filaments, and/or threads that are interlaid but not in an identifiable repeating manner as in a knitted or woven fabric. Nonwoven fabrics or webs, according to certain embodiments of the invention, may be formed by any process conventionally known in the art such as, for example, meltblowing processes, spunbonding processes, needle-punching, hydroentangling, air-laid, and bonded carded web processes. A "nonwoven web", as used herein, may comprise a plurality of individual fibers that have not been subjected to a consolidating process.

The terms "fabric" and "nonwoven fabric", as used herein, may comprise a web of fibers in which a plurality of the fibers are mechanically entangled or interconnected, fused together, and/or chemically bonded together. For example, a nonwoven web of individually laid fibers may be subjected to a bonding or consolidation process to bond at least a portion of the individually fibers together to form a coherent (e.g., united) web of interconnected fibers.

The term "consolidated" and "consolidation", as used herein, may comprise the bringing together of at least a portion of the fibers of a nonwoven web into closer proximity or attachment there-between (e.g., thermally fused together, chemically bonded together, and/or mechanically entangled together) to form a bonding site, or bonding sites, which function to increase the resistance to external forces (e.g., abrasion and tensile forces), as compared to the unconsolidated web. The bonding site or bonding sites, for example, may comprise a discrete or localized region of the web material that has been softened or melted and optionally subsequently or simultaneously compressed to form a discrete or localized deformation in the web material. Furthermore, the term "consolidated" may comprise an entire nonwoven web that has been processed such that at least a portion of the fibers are brought into closer proximity or attachment there-between (e.g., thermally fused together, chemically bonded together, and/or mechanically entangled together), such as by thermal bonding or mechanical entanglement (e.g., hydroentanglement) as merely a few examples. Such a web may be considered a "consolidated nonwoven", "nonwoven fabric" or simply as a "fabric" according to certain embodiments of the invention.

The term "staple fiber", as used herein, may comprise a cut fiber from a filament. In accordance with certain embodiments, any type of filament material may be used to form staple fibers. For example, staple fibers may be formed from polymeric fibers, and/or elastomeric fibers. Non-limiting examples of materials may comprise polyolefins (e.g., a polypropylene or polypropylene-containing copolymer), polyethylene terephthalate, and polyamides. The average length of staple fibers may comprise, by way of example only, from about 2 centimeter to about 15 centimeter.

The term "spunbond", as used herein, may comprise fibers which are formed by extruding molten thermoplastic material as filaments from a plurality of fine, usually circular, capillaries of a spinneret with the diameter of the extruded filaments then being rapidly reduced. According to an embodiment of the invention, spunbond fibers are generally not tacky when they are deposited onto a collecting surface and may be generally continuous as disclosed and described herein. It is noted that the spunbond used in certain composites of the invention may include a nonwoven described in the literature as SPINLACE^{®}. Spunbond fibers, for example, may comprises continuous fibers.

As used herein, the term "continuous fibers" refers to fibers which are not cut from their original length prior to being formed into a nonwoven web or nonwoven fabric. Continuous fibers may have average lengths ranging from greater than about 15 centimeters to more than one meter, and up to the length of the web or fabric being formed. For example, a continuous fiber, as used herein, may comprise a fiber in which the length of the fiber is at least 1,000 times larger than the average diameter of the fiber, such as the length of the fiber being at least about 5,000, 10,000, 50,000, or 100,000 times larger than the average diameter of the fiber.

The term "meltblown", as used herein, may comprise fibers formed by extruding a molten thermoplastic material through a plurality of fine die capillaries as molten threads or filaments into converging high velocity, usually hot, gas (e.g. air) streams which attenuate the filaments of molten thermoplastic material to reduce their diameter, which may be to microfiber diameter, according to certain embodiments of the invention. According to an embodiment of the invention, the die capillaries may be circular. Thereafter, the meltblown fibers are carried by the high velocity gas stream and are deposited on a collecting surface to form a web of randomly disbursed meltblown fibers. Meltblown fibers may comprise microfibers which may be continuous or discontinuous and are generally tacky when deposited onto a collecting surface. Meltblown fibers, however, are shorter in length than those of spunbond fibers.

As used herein, the term "monolithic" film may comprise any film that is continuous and substantially free or free of pores (e.g., devoid of pores). In certain alternative embodiments of the invention, a "monolithic" film may comprise fewer pore structures than would otherwise be found in a microporous film. According to certain non-limiting example embodiments of the invention, a monolithic film may act as a barrier to liquids and particulate matter but allow water vapor to pass through. In addition, without intending to be bound by theory, by achieving and maintaining high breathability, it is possible to provide an article that is more comfortable to wear because the migration of water vapor through the laminate helps reduce and/or limit discomfort resulting from excess moisture trapped against the skin. A "monolithic" film, for example, may comprise a highly breathable polymer.

The term "highly breathable polymer", as used herein, may comprise any polymer or elastomer that is selectively permeable to water vapor but substantially impermeable to liquid water and that can form a breathable film, for example, in which the polymer is capable of absorbing and desorbing water vapor and providing a barrier to aqueous fluids (e.g., water, blood, etc.). For example, a highly breathable polymer can absorb water vapor from one side of a film and release it to the other side of film, thereby allowing the water vapor to be transported through the film. As the highly breathable polymer can impart breathability to films, films formed from such polymers do not need to include pores (e.g., monolithic film). According to certain embodiments of the invention, "highly breathable polymer" may comprise any thermoplastic polymer or elastomer having a moisture vapor transmission rate (MVTR) of at least 500 g/m²/day when formed into a film. According to certain embodiments of the invention, "highly breathable polymer" may comprise any thermoplastic polymer or elastomer having a MVTR of at least 750 g/m²/day or of at least 1000 g/m²/day when formed into a film, such as a film having, for example, a thickness of about 25 microns or less. According to certain embodiments of the invention, highly breathable polymers may comprise, for example, any one or combination of a polyether block amide copolymer (e.g., PEBAX^{®} from Arkema Group), polyester block amide copolymer, copolyester thermoplastic elastomer (e.g., ARNITEL^{®} from DSM Engineering Plastics, HYTREL^{®} from E.I. DuPont de Nemours and Company), or thermoplastic urethane elastomer (TPU).

The term "microporous" film, as used herein, may comprise a polymeric film layer hiving a plurality of micropores dispersed throughout a body of the film. Microporous films, for example, may generally be produced by dispersing finely divided particles of a non-hygroscopic filler material, such as an inorganic salt (e.g., calcium carbonate), into a suitable polymer followed by forming a film of the filled polymer and stretching the film to provide good porosity and water vapor absorption or transmission. For example, microporous film breathability may be dependent on the formation of a tortuous porous path throughout the film via the stretching of the filler impregnated film to impart the desired porosity (e.g., pore formation). Furthermore, the barrier properties of such microporous films are affected by the surface tension of the liquid to which they are exposed (e.g., they are more easily penetrated by isopropyl alcohol than by water), and they transmit odor more easily than solid films (e.g., monolithic films).

The term "layer", as used herein, may comprise a generally recognizable combination of similar material types and/or functions existing in the X-Y plane.

All whole number end points disclosed herein that can create a smaller range within a given range disclosed herein are within the scope of certain embodiments of the invention. By way of example, a disclosure of from about 10 to about 15 includes the disclosure of intermediate ranges, for example, of: from about 10 to about 11; from about 10 to about 12; from about 13 to about 15; from about 14 to about 15; etc. Moreover, all single decimal (e.g., numbers reported to the nearest tenth) end points that can create a smaller range within a given range disclosed herein are within the scope of certain embodiments of the invention. By way of example, a disclosure of from about 1.5 to about 2.0 includes the disclosure of intermediate ranges, for example, of: from about 1.5 to about 1.6; from about 1.5 to about 1.7; from about 1.7 to about 1.8; etc.

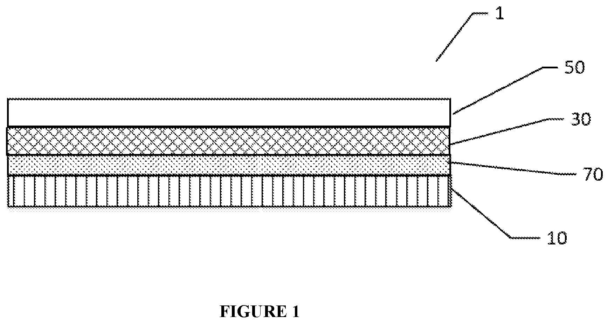

In one aspect, the present invention provides a warming blanket, such as for use outside and/or during the nighttime, including (i) a perspiration absorptive layer (PAL); (ii) a metal coating layer (MCL); and (iii) a transparent coating layer (TCL); wherein the MCL is located directly or indirectly between the PAL and the TCL. Figure 1, for instance, illustrates a warming blanket 1 including a PAL 10, a MCL 30, and a TCL 50, in which the MCL is located between the PAL and the TCL. As shown in Figure 1, the TCL 50 may be adjacent and in contact with the MCL, while a first adhesive layer 70 may be disposed between and bond the PAL to the MCL.

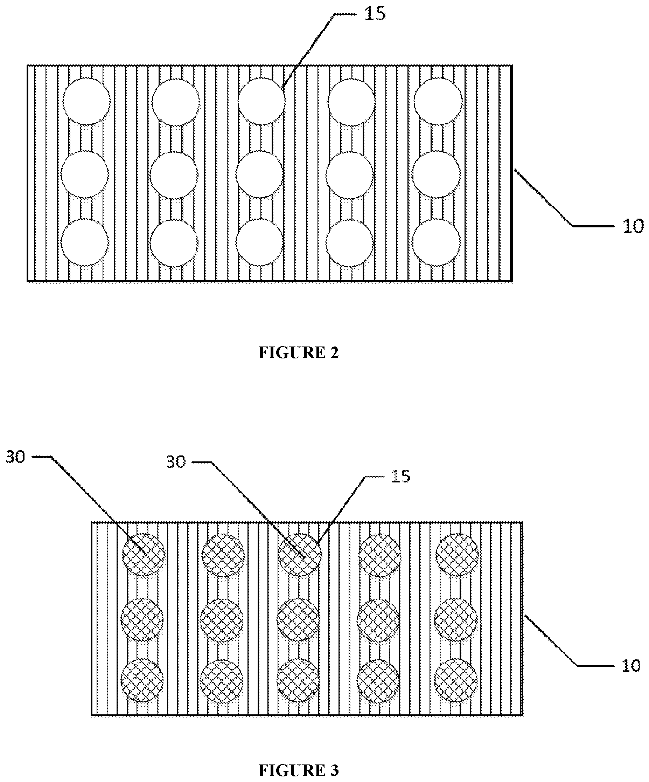

In accordance with certain embodiments of the invention, the PAL comprises a woven fabric or a nonwoven fabric. As noted above, according to the invention, the PAL comprises a plurality of through-holes formed through a total thickness of the PAL in a z-direction that is perpendicular to an x-y plane of the PAL. Figure 2, for instance, illustrates a PAL 10 including a plurality of through-holes 15 that extend completely through the entire thickness of the PAL. Meanwhile, Figure 3 illustrates the PAL 10 of Figure 2 overlying a MCL 30 that is visible through the plurality of through-holes 15, in accordance with certain embodiments of the invention.

The plurality of through-holes, in accordance with certain embodiments of the invention, may have an average individual open area from about 1 mm² to about 100 mm², such as at least about any of the following: 1, 3, 5, 8, 10, 15, 20, 25, 30, 35, 40, 45, and 50 mm², and/or at most about any of the following: 100, 95, 90, 85, 80, 75, 70, 65, 60, 55, and 50 mm². In accordance with certain embodiments of the invention, the PAL may include particularly smaller through-holes similar to that of a cross-stitching fabric (e.g., an average individual open area near the lower end of the ranges set forth above). Additionally or alternatively, the PAL may include through-holes having a more macroscopic nature (e.g., an average individual open area near the upper end of the ranges set forth above), in which the through-holes may be formed or cut-out after formation of the fabric. In certain embodiments of the invention, the PAL may comprises a hydroentangled nonwoven fabric, in which the through-holes may have a more macroscopic nature that were formed during a hydroentanglement operation. Additionally or alternatively, the plurality of through-holes may define a total open area from about 10 to about 80%, such as at least about any of the following: 10, 15, 20, 25, 30, 35, 40, 45, and 50%, and/or at most about any of the following: 80, 75, 70, 65, 60, 55, and 50% (e.g., 40% to 60%).

In accordance with certain embodiments of the invention, the PAL may comprise a gridding fabric, such as a woven gridding fabric or a nonwoven gridding fabric. Additionally or alternatively, the PAL may comprise one or more spunbond layers, one or more meltblown layers, one or more cellulose-containing layers, one or more needlepunched layers, one or more hydroentangled layers, one or more carded staple fiber layers, one or more air-laid layers, one or more sub-micron layers, or any combinations thereof. Additionally or alternatively, the PAL may comprise a synthetic polymer, such as one or more polyolefins, one or more polyesters, one or more polyamides, or any combination thereof. Additionally or alternatively, the PAL may comprise a natural cellulosic material, a synthetic cellulosic material, or any combination thereof, such as cotton, pulp, viscose, and rayon. Additionally or alternatively, the PAL may comprise a plurality of superabsorbent polymer (SAP) components, such as beads or particulates, embedded within a body portion of the PAL. For example, the SAP components may be housed or entangled within a plurality of the fibers (e.g., synthetic and/or cellulosic). Additionally or alternatively, the PAL may be provided as a nonwoven web (e.g., non-consolidated) or as a nonwoven fabric that has been consolidated by any means disclosed herein. For example, the PAL may be consolidated by thermal calendering, ultrasonic bonding, mechanical bonding (e.g., hydroentangling), chemical bonding, or any combination thereof).

In accordance with certain embodiments of the invention, the PAL may comprise a spunbond-meltblown-spunbond structure or a spunbond-cellulose-spunbond structure. The PAL, in accordance with certain embodiments of the invention, may comprise a hydroentangled composite formed from a first spunbond layer, a first cellulose-containing layer, and a second spunbond layer. For example, the plurality of through-holes of the PAL may be formed during a hydroentanglement operation.

In accordance with certain embodiments of the invention, the PAL may have a basis weight from 5 to about 500 gsm, such as at least about any of the following: 5, 6, 8, 10, 12, 15, 25, 50, 75, 100, 150, 200, and 250 gsm, and/or at most about any of the following: 500, 450, 400, 350, 300, and 250 gsm.

In accordance with the invention, the warming blanket includes a first adhesive layer located between and bonding the PAL and the MCL. The first adhesive layer, for example, may comprise a first discontinuous pattern, in which the first discontinuous pattern comprises a first plurality of discrete islands of adhesive surrounded by regions devoid of adhesive. Alternatively, the first adhesive layer may comprise a first discontinuous pattern, in which the first discontinuous pattern comprises a first plurality of discrete islands that are devoid of adhesive and surrounded by regions of adhesive. Alternatively, the first adhesive layer may comprise a first discontinuous pattern, in which the first discontinuous pattern comprises a first plurality of separate and distinct lines of adhesive, and wherein the first plurality of separate and distinct lines of adhesive may be straight, arcuate, or have a zig-zag configuration.

The first discontinuous pattern, in accordance with certain embodiments of the invention, may comprise regions that are devoid of adhesive that are aligned, at least partially, with the plurality of through-holes of the PAL. For example, the first discontinuous pattern may overlap no more than about 50% of the total open area of the PAL, such as at least about any of the following: 0, 3, 5, 8, 10, 12, 15, 18, 20, 22, and 25%, and/or at most about any of the following: 50, 45, 40, 35, 30, 28, 26, and 25%.

In accordance with certain embodiments of the invention, the first adhesive layer may have a basis weight from about 0.2 to about 5 gsm, such as at least about any of the following: 0.25, 0.5, 0,75, 1, 1.5, 2 and 2.5 gsm, and/or at most about any of the following: 5, 4, 3, and 2.5 gsm. Additionally or alternatively, the first adhesive layer, in accordance with certain embodiments of the invention, may comprise a moisture-proof pressure sensitive adhesive, an acrylic holt melt adhesive, or combinations thereof.

In accordance with certain embodiments of the invention, the MCL may comprise a highly reflective metal or highly reflective metal alloy. For example, the highly reflective metal or highly reflective metal alloy may reflect at least about 80% of electromagnetic radiation across all wavelengths from about 1 to about 20 microns, such as across all wavelengths from about 8 to about 15 microns; or such as at least about 85%, or at least about 90%, or at least about 95% of electromagnetic radiation across all wavelengths from about 1 to about 20 microns, such as across all wavelengths from about 8 to about 15 microns. Additionally or alternatively, the highly reflective metal or highly reflective metal alloy may comprise aluminum or an alloy thereof, gold or an alloy thereof, copper or an alloy thereof, silver or an alloy thereof, or any combination thereof. Additionally or alternatively, the MCL may have an average thickness from about 100 nm to about 1,000 nm, such as at least about any of the following: 100, 200, 300, 400, and 500 nm, and/or at most about any of the following: 1000, 900, 800, 700, 600, and 500 nm. Additionally or alternatively, the MCL may have been formed by a vacuum coating method, such as by thermal evaporation, E-beam evaporation, sputtering, arc ion plating, plasma enhanced chemical vapor deposition, or atomic layer deposition.

In accordance with certain embodiments of the invention, the TCL may be directly adjacent the MCL. In this regard, the TCL may be provided and formed, while the MCL may be deposited or otherwise formed directly onto the TCL. The TCL, in accordance with certain embodiments of the invention, may be at least 75% transparent, such as at least 80%, 85%, 90%, 95%, or 99% transparent, to electromagnetic radiation across all wavelengths from about 0.1 to about 0.4 microns. Additionally or alternatively, the TCL may be at least 75% transparent, such as at least 80%, 85%, 90%, 95%, or 99% transparent, to electromagnetic radiation across all wavelengths from about 0.4 to about 0.7 microns. Additionally or alternatively, the TCL may be at least 75% transparent, such as at least 80%, 85%, 90%, 95%, or 99% transparent, to electromagnetic radiation across all wavelengths from about 0.7 to about 1000 microns.

The TCL, in accordance with certain embodiments of the invention, may comprise a polypropylene, a polyethylene, a polyester, such as a polyethylene terephthalate, a thermoplastic elastomer, a thermoplastic polyurethane, a polybutylene terephthalate, polybutylene adipate terephthalate, a polybutyrate, a polylactic acid, or any combinations thereof. By way of example only, the TCL may comprise a polyethylene film having a thickness from 0.10 - 0.12 mm, in which sunlight is around 90% transparent. Additionally or alternatively, the TCL may comprise an anti-reflective coating and defines a first outermost surface of the warming blanket. In accordance with certain embodiments of the invention, the anti-reflective coating, for example, may be a single layer or multiple layers depending on the particular material or materials of construction and transparency desired. By way of example only, the TCL may include a nano-array coating, uniform continuous coating, or a mesoporous structure coating. The coating method, by way of example only, may be an evaporation process, a sputtering process, a roll coating process, as well as other suitable processes for providing a coating to form the TCL. Additionally or alternatively, the TCL may have a thickness from about 5 to about 150 microns, such as at least about any of the following: 5, 10, 15, 20, 25, 30, 40, 50, 60, 70, and 75 microns, and/or at most about any of the following: 150, 125, 100, 90, 80, and 75 microns.

In accordance with certain embodiments of the invention, the TCL may be a film comprising a single layer microporous film or a single layer monolithic film. Alternatively, the film may comprise a multilayer film including one or more microporous films and/or one or more monolithic films.

In accordance with certain embodiments of the invention, the TCL may have a moisture vapor transmission rate (MVTR) of at least about 25 g/m² per 24 hours as determined by ASTM E96D, such as at least about any of the following: 25, 50, 75, 100, 125, 150, 175, and 200 g/m² per 24 hours as determined by ASTM E96D, and/or at most about any of the following: 500, 450, 400, 350, 300, 275, 250, 225, and 200 g/m² per 24 hours as determined by ASTM E96D. Additionally or alternatively, the TCL may have a hydrostatic head (HSH) of at least about 50 mbar as determined by AATCC 127 (60 mbar/min), such as at least about any of the following: 50, 60, 75, 80, 100, and 125 mbar as determined by AATCC 127 (60 mbar/min), and/or at most about any of the following: 200, 175, 150, and 125 mbar as determined by AATCC 127 (60 mbar/min).

In accordance with certain embodiments of the invention, the warming blanket may have a moisture vapor transmission rate (MVTR) of at least about 25 g/m² per 24 hours as determined by ASTM E96D, such as at least about any of the following: 25, 50, 75, 100, 125, 150, 175, and 200 g/m² per 24 hours as determined by ASTM E96D, and/or at most about any of the following: 500, 450, 400, 350, 300, 275, 250, 225, and 200 g/m² per 24 hours as determined by ASTM E96D. Additionally or alternatively, the warming blanket may have a hydrostatic head (HSH) of at least about 50 mbar as determined by AATCC 127 (60 mbar/min), such as at least about any of the following: 50, 60, 75, 80, 100, and 125 mbar as determined by AATCC 127 (60 mbar/min), and/or at most about any of the following: 200, 175, 150, and 125 mbar as determined by AATCC 127 (60 mbar/min).

In another aspect, the present invention provides a method of producing a method of producing a warming blanket, such as those described and disclosed herein. The method may comprise the following: (i) providing a transparent coating layer (TCL); (ii) depositing a metal coating layer (MCL) directly onto the TCL; (iii) providing or forming a perspiration absorptive layer (PAL), which may include a plurality of through-holes as described above; and (iv) bonding the PAL to the MCL to provide the warming blanket, such as those described and disclosed herein.

In accordance with certain embodiments of the invention, the step of bonding the PAL to the MCL may comprise adhesively bonding the PAL directly to the MCL via a first adhesive layer, as noted above. Additionally or alternatively, the first adhesive layer may be deposited onto the PAL, followed by lamination of the PAL to the MCL, in which the first adhesive layer is located between and adjacent the PAL and the MCL. Additionally or alternatively, the first adhesive layer may be deposited onto the MCL, followed by lamination of the PAL and the MCL, in which the first adhesive layer is located between and adjacent the PAL and the MCL. The first adhesive layer, as described above, may comprise a discontinuous pattern.

These and other modifications and variations to the invention may be practiced by those of ordinary skill in the art without departing from the scope of the invention, which is more particularly set forth in the appended claims. In addition, it should be understood that aspects of the various embodiments may be interchanged in whole or in part. Furthermore, those of ordinary skill in the art will appreciate that the foregoing description is by way of example only, and it is not intended to limit the invention as further described in such appended claims. Therefore, the scope of the appended claims should not be limited to the exemplary description of the versions contained herein.

## Claims

1. A warming blanket, comprising:
(i) a perspiration absorptive layer (PAL) (10) comprising a woven fabric or a nonwoven fabric, wherein the PAL comprises a plurality of through-holes (15) formed through a total thickness of the PAL in a z-direction that is perpendicular to an x-y plane of the PAL;
(ii) a metal coating layer (MCL) (30);
(iii) a first adhesive layer (70) located between and bonding the PAL and the MCL, wherein the first adhesive layer comprises a first discontinuous pattern including regions that are devoid of adhesive that are at least partially aligned with the plurality of through-holes of the PAL; and
(iv) a transparent coating layer (TCL) (50); wherein the MCL is located directly or indirectly between the PAL and the TCL.

2. The warming blanket of claim 1, wherein (i) the plurality of through-holes have an average individual open area from about 1 mm² to about 100 mm²; (ii) the plurality of through-holes define a total open area from about 10 to about 80%; or (iii) both (i) and (ii).

3. The warming blanket of claim 1 or 2, wherein the first adhesive layer has a basis weight from 0.2 to 5 grams-per-square meter (gsm).

4. The warming blanket of any of claims 1 to 3, wherein the first discontinuous pattern comprises a first plurality of discrete islands of adhesive surrounded by regions devoid of adhesive.

5. The warming blanket of any of claims 1 to 4, wherein the first discontinuous pattern comprises a first plurality of discrete islands that are devoid of adhesive and surrounded by regions of adhesive.

6. The warming blanket of any of claims 1 to 5, wherein the first discontinuous pattern comprises a first plurality of separate and distinct lines of adhesive, and wherein the first plurality of separate and distinct lines of adhesive may be straight, arcuate, or have a zig-zag configuration.

7. The warming blanket of any of claims 1 to 6, wherein the first discontinuous pattern overlaps no more than about 50% of the total open area of the PAL.

8. The warming blanket of any of claims 1 to 7, wherein the MCL comprises a highly reflective metal or highly reflective metal alloy.

9. The warming blanket of claim 8, wherein the highly reflective metal or highly reflective metal alloy reflects at least about 80% of electromagnetic radiation across all wavelengths from about 1 to about 20 microns.

10. The warming blanket of claim 8 or 9, wherein the highly reflective metal or highly reflective metal alloy comprises aluminum or an alloy thereof, gold or an alloy thereof, copper or an alloy thereof, silver or an alloy thereof, or any combination thereof.

11. The warming blanket of any of claims 1 to 10, wherein the TCL is directly adjacent the MCL, and optionally comprises a polyethylene film.

12. The warming blanket of any of claims 1 to 11, wherein the TCL is at least 75% transparent to electromagnetic radiation across all wavelengths from about 0.1 to about 0.4 microns.

13. The warming blanket of any of claims 1 to 12, wherein the TCL is at least 75% transparent to electromagnetic radiation across all wavelengths from about 0.4 to about 0.7 microns.

14. The warming blanket of any of claims 1 to 13, wherein the TCL is at least 75% transparent to electromagnetic radiation across all wavelengths from about 0.7 to about 1000 microns.

15. A method of producing a warming blanket according to claim 1, comprising:
(i) providing a transparent coating layer (TCL)(50);
(ii) depositing a metal coating layer (MCL) (30) directly onto the TCL:
(iii) providing or forming a perspiration absorptive layer (PAL) (10) comprising a woven fabric or a non-woven fabric, wherein the PAL comprises a plurality of through-holes formed through a total thickness of the PAL in a z-direction that is perpendicular to an x-y plane of the PAL; and
(iv) adhesively bonding the PAL to the MCL using a first adhesive layer (70) located between and bonding the PAL and the MCL, wherein the first adhesive layer comprises a first discontinuous pattern including regions that are devoid of adhesive that are at least partially aligned with the plurality of through-holes of the PAL, to provide the warming blanket.

## Patentansprüche

1. Eine Wärmedecke, bestehend aus:
(i) einer Schweißabsorbierenden Schicht (perspiration absorptive layer; PAL) (10), die ein Gewebe oder ein Vlies umfasst, wobei die PAL eine Vielzahl von Durchgangslöchern (15) umfasst, die durch die gesamte Dicke der PAL in einer z-Richtung gebildet sind, die senkrecht zu einer x-y-Ebene der PAL ist;
(ii) eine Metallbeschichtungsschicht (metal coating layer; MCL) (30);
(iii) eine erste Klebeschicht (70), die sich zwischen der PAL und der MCL befindet und diese verbindet, wobei die erste Klebeschicht ein erstes diskontinuierliches Muster umfasst, das Bereiche ohne Klebstoff enthält, die zumindest teilweise mit den mehreren Durchgangslöchern der PAL ausgerichtet sind; und
(iv) eine transparente Beschichtungsschicht (transparent coating layer; TCL) (50); wobei sich die MCL direkt oder indirekt zwischen der PAL und der TCL befindet.

2. Wärmedecke nach Anspruch 1, wobei (i) die Vielzahl von Durchgangslöchern eine durchschnittliche individuelle offene Fläche von etwa 1 mm² bis etwa 100 mm² aufweisen; (ii) die Vielzahl von Durchgangslöchern eine gesamte offene Fläche von etwa 10 bis etwa 80 % definieren; oder (iii) sowohl (i) als auch (ii).

3. Wärmedecke nach Anspruch 1 oder 2, wobei die erste Klebeschicht ein Flächengewicht von 0,2 bis 5 Gramm pro Quadratmeter (g/m²) aufweist.

4. Wärmedecke nach einem der Ansprüche 1 bis 3, wobei das erste diskontinuierliche Muster eine erste Vielzahl von diskreten Klebstoffinseln umfasst, die von klebstofffreien Bereichen umgeben sind.

5. Wärmedecke nach einem der Ansprüche 1 bis 4, wobei das erste diskontinuierliche Muster eine erste Vielzahl von diskreten Inseln umfasst, die frei von Klebstoff sind und von Klebstoffbereichen umgeben sind.

6. Wärmedecke nach einem der Ansprüche 1 bis 5, wobei das erste diskontinuierliche Muster eine erste Vielzahl von getrennten und unterschiedlichen Klebstofflinien umfasst und wobei die erste Vielzahl von getrennten und unterschiedlichen Klebstofflinien gerade, bogenförmig oder zickzackförmig sein kann.

7. Wärmedecke nach einem der Ansprüche 1 bis 6, wobei das erste diskontinuierliche Muster nicht mehr als etwa 50 % der gesamten offenen Fläche der PAL überlappt.

8. Wärmedecke nach einem der Ansprüche 1 bis 7, wobei die MCL ein hochreflektierendes Metall oder eine hochreflektierende Metalllegierung umfasst.

9. Wärmedecke nach Anspruch 8, wobei das hochreflektierende Metall oder die hochreflektierende Metalllegierung mindestens etwa 80 % der elektromagnetischen Strahlung über alle Wellenlängen von etwa 1 bis etwa 20 Mikrometern reflektiert.

10. Wärmedecke nach Anspruch 8 oder 9, wobei das hochreflektierende Metall oder die hochreflektierende Metalllegierung Aluminium oder eine Legierung davon, Gold oder eine Legierung davon, Kupfer oder eine Legierung davon, Silber oder eine Legierung davon oder eine beliebige Kombination davon umfasst.

11. Wärmedecke nach einem der Ansprüche 1 bis 10, wobei die TCL direkt an die MCL angrenzt und optional eine Polyethylenfolie umfasst.

12. Wärmedecke nach einem der Ansprüche 1 bis 11, wobei die TCL für elektromagnetische Strahlung über alle Wellenlängen von etwa 0,1 bis etwa 0,4 Mikrometer zu mindestens 75 % transparent ist.

13. Wärmedecke nach einem der Ansprüche 1 bis 12, wobei die TCL für elektromagnetische Strahlung über alle Wellenlängen von etwa 0,4 bis etwa 0,7 Mikrometer zu mindestens 75 % transparent ist.

14. Wärmedecke nach einem der Ansprüche 1 bis 13, wobei die TCL für elektromagnetische Strahlung über alle Wellenlängen von etwa 0,7 bis etwa 1000 Mikrometer zu mindestens 75 % transparent ist.

15. Verfahren zur Herstellung einer Wärmedecke gemäß Anspruch 1, umfassend:
(i) Bereitstellen einer transparenten Beschichtungsschicht (TCL) (50);
(ii) Aufbringen einer Metallbeschichtungsschicht (MCL) (30) direkt auf die TCL;
(iii) Bereitstellen oder Ausbilden einer schweißabsorbierenden Schicht (PAL) (10), die ein Gewebe oder Vlies umfasst, wobei die PAL eine Vielzahl von Durchgangslöchern umfasst, die durch die gesamte Dicke der PAL in einer z-Richtung ausgebildet sind, die senkrecht zu einer x-y-Ebene der PAL ist; und
(iv) Verbinden der PAL mit der MCL unter Verwendung einer ersten Klebeschicht (70), die sich zwischen der PAL und der MCL befindet und diese verbindet, wobei die erste Klebeschicht ein erstes diskontinuierliches Muster umfasst, das Bereiche ohne Klebstoff umfasst, die zumindest teilweise mit der Vielzahl von Durchgangslöchern der PAL ausgerichtet sind, um die Wärmedecke bereitzustellen.

## Revendications

1. Couverture chauffante, comprenant :
(i) une couche absorbant la transpiration (PAL) (10) comprenant un tissu tissé ou un tissu non tissé, dans laquelle la PAL comprend une pluralité de trous traversants (15) formés à travers l'épaisseur totale de la PAL dans une direction z qui est perpendiculaire à un plan x-y de la PAL ;
(ii) une couche de revêtement métallique (MCL) (30) ;
(iii) une première couche adhésive (70) située entre la PAL et la MCL et les liant entre elles, dans laquelle la première couche adhésive comprend un premier motif discontinu comprenant des régions dépourvues d'adhésif qui sont au moins partiellement alignées avec la pluralité de trous traversants de la PAL ; et
(iv) une couche de revêtement transparente (TCL) (50) ; dans laquelle la MCL est située directement ou indirectement entre le PAL et la TCL.

2. La couverture chauffante selon la revendication 1, dans laquelle (i) la pluralité de trous traversants ont une surface ouverte individuelle moyenne ^{comprise} entre environ 1 ^{mm²} et environ 100 ^{mm²} ; (ii) la pluralité de trous traversants définissent une surface ouverte totale comprise entre environ 10 et environ 80 % ; ou (iii) à la fois (i) et (ii).

3. La couverture chauffante selon la revendication 1 ou 2, dans laquelle la première couche adhésive a un poids de base compris entre 0,2 et 5 grammes par mètre carré (g/m²).

4. La couverture chauffante selon l'une quelconque des revendications 1 à 3, dans laquelle le premier motif discontinu comprend une première pluralité d'îlots discrets d'adhésif entourés de régions dépourvues d'adhésif.

5. La couverture chauffante selon l'une quelconque des revendications 1 à 4, dans laquelle le premier motif discontinu comprend une première pluralité d'îlots discrets dépourvus d'adhésif et entourés de zones d'adhésif.

6. La couverture chauffante selon l'une quelconque des revendications 1 à 5, dans laquelle le premier motif discontinu comprend une première pluralité de lignes d'adhésif séparées et distinctes, et dans laquelle la première pluralité de lignes d'adhésif séparées et distinctes peut être droite, arquée ou avoir une configuration en zigzag.

7. La couverture chauffante selon l'une quelconque des revendications 1 à 6, dans laquelle le premier motif discontinu ne recouvre pas plus d'environ 50 % de la surface ouverte totale du PAL.

8. La couverture chauffante selon l'une quelconque des revendications 1 à 7, dans laquelle le MCL comprend un métal hautement réfléchissant ou un alliage métallique hautement réfléchissant.

9. La couverture chauffante selon la revendication 8, dans laquelle le métal hautement réfléchissant ou l'alliage métallique hautement réfléchissant réfléchit au moins environ 80 % du rayonnement électromagnétique sur toutes les longueurs d'onde comprises entre environ 1 et environ 20 microns.

10. Couverture chauffante selon la revendication 8 ou 9, dans laquelle le métal hautement réfléchissant ou l'alliage métallique hautement réfléchissant comprend de l'aluminium ou un alliage de celui-ci, de l'or ou un alliage de celui-ci, du cuivre ou un alliage de celui-ci, de l'argent ou un alliage de celui-ci, ou toute combinaison de ceux-ci.

11. La couverture chauffante selon l'une quelconque des revendications 1 à 10, dans laquelle le TCL est directement adjacent au MCL et comprend éventuellement un film de polyéthylène.

12. Couverture chauffante selon l'une quelconque des revendications 1 à 11, dans laquelle le TCL est au moins 75 % transparent au rayonnement électromagnétique sur toutes les longueurs d'onde comprises entre environ 0,1 et environ 0,4 micron.

13. Couverture chauffante selon l'une quelconque des revendications 1 à 12, dans laquelle le TCL est transparent à au moins 75 % au rayonnement électromagnétique sur toutes les longueurs d'onde comprises entre environ 0,4 et environ 0,7 micron.

14. La couverture chauffante selon l'une quelconque des revendications 1 à 13, dans laquelle le TCL est transparent à au moins 75 % au rayonnement électromagnétique sur toutes les longueurs d'onde comprises entre environ 0,7 et environ 1 000 microns.

15. Procédé de fabrication d'une couverture chauffante selon la revendication 1, comprenant :
(i) la fourniture d'une couche de revêtement transparente (TCL) (50) ;
(ii) le dépôt d'une couche de revêtement métallique (MCL) (30) directement sur la TCL ;
(iii) la fourniture ou la formation d'une couche absorbant la transpiration (PAL) (10) comprenant un tissu tissé ou non tissé, dans laquelle la PAL comprend une pluralité de trous traversants formés à travers l'épaisseur totale de la PAL dans une direction z qui est perpendiculaire à un plan x-y de la PAL ; et
(iv) coller la PAL à la MCL à l'aide d'une première couche adhésive (70) située entre la PAL et la MCL et les collant l'une à l'autre, la première couche adhésive comprenant un premier motif discontinu comprenant des zones dépourvues d'adhésif qui sont au moins partiellement alignées avec la pluralité de trous traversants de la PAL, afin de fournir la couverture chauffante.
